# EUROPEAN PATENT APPLICATION

(11) **EP 4 606 381 A1**
(43) Date of publication of application: **27.08.2025**
(21) Application number: 23879163.6
(22) Date of filing: 19.10.2023
(51) Int. Cl.: A61K 35/57, A61K 35/50, A61K 45/08, A61P 1/00, A61P 3/00, A61P 9/00, A61P 11/00, A61P 13/00, A61P 17/00, A61P 19/00, A61P 25/00, A61P 27/00, A61P 31/00, A61P 35/00, A61P 37/00, A61P 43/00

(54) **DRUG FOR TREATING OR PREVENTING MACROPHAGE-MEDIATED DISEASES AND USE THEREOF**

(30) Priority: 20.10.2022 CN 202211286565
(71) Applicant: Anhui Hygeiancells Biomedical Co. Ltd, Huangshan City, Anhui 245021 (CN)
(72) Inventor: QIAN, Jin, Huangshan, Anhui 245021 (CN)
(74) Representative: Delbarba, Andrea
(86) International application number: PCT/CN2023/125342
(87) International publication number: WO 2024/083176

(57) **Abstract**

Disclosed are the uses of amniotic fluid in the preparation of a drug for treating and/or preventing macrophage-mediated diseases, a drug for treating and/or preventing type M1 macrophage-mediated diseases, a drug for treating and/or preventing type M2 macrophage-mediated diseases, a preparation for treating and/or preventing TLR4/NF-κB signaling pathway-mediated diseases, a preparation for inhibiting the number of type M1 macrophages and increasing the proportion of type M2 macrophages or a preparation for promoting the conversion of type M1 macrophages to type M2 macrophages. The amniotic fluid is from eggs at an embryo age of 5-12 days, or eggs from poultry other than chickens at the development stage corresponding to the development stage of the above-mentioned eggs at the embryo age; or embryos of rodent animals at the embryo age of 8-14 days, or embryos of non-human mammals other than rodent animals at the development stage corresponding to the development stage of the above-mentioned rodent animals at the embryo age of 8-14 days.

## Description

### FIELD OF THE INVENTION

The present invention relates to medicines for treating or preventing macrophage-mediated diseases and applications thereof.

### BACKGROUND OF THE INVENTION

Macrophages manifest as different types in response to various stimulations, inducing higher expression levels of inducible nitric oxide synthase (iNOS) or arginase, and are accordingly classified as M1 and M2 macrophages. In inflammatory diseases, M1 macrophages accumulate at early inflammatory sites and are activated by pro-inflammatory cytokines such as LPS (lipopolysaccharide), TNFα (tumor necrosis factor alpha), and IFNγ (interferon gamma). They subsequently promote the occurrence and progression of inflammation by secreting inflammatory factors such as IL12, protecting the body from invasion by foreign substances. In the late stage of inflammation, M2 macrophages play roles in inhibiting inflammation, repairing tissues, and reconstructing tissue structures. During the occurrence and progression of inflammation, the ratio between M1 and M2 macrophage populations continuously changes over time, eventually eliminating the effects of inflammation completely. However, in certain chronic inflammations and specific acute inflammatory processes, imbalance in the ratio between M1 and M2 macrophage populations occurs. Hyperactivation of M1 macrophage populations can lead to severe tissue damage and subsequent adverse symptoms such as more severe inflammatory cytokine storms. Therefore, M2 macrophages are closely associated with anti-inflammatory responses and immune homeostasis. They participate in tissue repair, tissue and organ remodeling, scar formation, and wound healing processes, exhibiting immunosuppressive effects.

Various types of miRNAs, transcription factors, and other non-coding RNAs derived from exosomes regulate M2 macrophage polarization through different signaling pathways; wherein, the Toll-like receptor 4 (TLR4)/NF-xB signaling pathway plays a central regulatory role in inflammatory responses. In the non-activated state, NF-κB activity is inhibited by IκB; upon stimulation by TLRs and cytokine receptors including TNF receptors, activation of IκB kinase induces IκB phosphorylation, further causing proteasomal degradation and subsequent release of NF-κB for translocation toward the nucleus, and activating the transcription of target genes. The activation of NF-κB can contribute to the synthesis and release of inflammatory cytokines.

With the increasing incidence of diabetes, diabetic ulcers have become one of the most common chronic refractory wounds (CRW) in clinical practice, with persistent chronic inflammation as a typical characteristic of diabetic skin wounds. Due to the complex micro environments of diabetic wounds, such as hypoxia, infection, ischemic state, inflammation, and oxidative stress, diabetic ulcers often become prolonged and recurrent, while traditional debridement and dressing therapies exert limited effects on diabetic wounds. Therefore, how to effectively and economically treat diabetic ulcers has become an urgent problem to be solved.

In the wound healing process, M1 macrophages are responsible for phagocytosing necrotic tissues and cellular debris, while M2 macrophages participate in inhibiting inflammation and promoting tissue regeneration. Diabetic wounds are typically in an excessive inflammatory state, with a large number of M1 macrophages present in the wound tissue, thus hindering the transformation into M2 macrophages. Notably, previous studies have indicated that increasing the M2 phenotype may be an important factor in the repair of diabetic wounds.

### SUMMARY OF THE INVENTION

In a first aspect, the present invention provides use of amniotic fluid in the preparation of medicines for treating and/or preventing macrophage-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In a second aspect, the present invention provides use of amniotic fluid in the preparation of medicines for treating and/or preventing M1 macrophage-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In a third aspect, the present invention provides use of amniotic fluid in the preparation of medicines for treating and/or preventing M2 macrophage-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In a fourth aspect, the present invention provides use of amniotic fluid in the preparation of formulations for treating and/or preventing TLR4/NF-κB signaling pathway-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In a fifth aspect, the present invention provides use of amniotic fluid in the preparation of formulations for inhibiting the number of M1 macrophage populations and increasing the proportion of M2 macrophages, or in the preparation of formulations for promoting the transformation of M1 macrophages into M2 macrophages; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In one or more embodiments, the medicine or formulation is a cell culture containing the amniotic fluid and/or embryonic stem cells.

In one or more embodiments, the medicine or formulation is a pharmaceutical composition containing the amniotic fluid and/or chicken embryonic stem cells along with pharmaceutically acceptable excipients.

In one or more embodiments, the macrophage-mediated diseases are selected from: hypertrophic scars; chronic obstructive pneumonia; tumors, such as breast cancer, liver cancer; metabolic diseases, such as severe obesity, insulin resistance, type II diabetes; inflammatory diseases, such as acute pancreatitis, atherosclerosis; cardiovascular diseases, such as myocarditis, myocardial infarction, cardiac arrhythmia; neuropathies, such as Alzheimer's disease; cerebral diseases, such as cerebral infarction, cerebral injury; ophthalmic diseases, such as autoimmune uveitis, retinopathy, keratitis, corneal transplantation, sicca syndrome, uveal melanoma, myopia; immune inflammatory diseases, such as inflammatory enteritis, autoimmune hepatitis, asthma, alcoholic liver disease, colitis, multiple sclerosis, periodontitis; arthritis, such as rheumatoid arthritis, bone erosion, synovitis, osteoarthritis; nephritides, such as acute kidney injury, chronic kidney disease, end-stage renal disease, proliferative glomerulonephritis, membranous nephropathy, diabetic nephropathy, Henoch-Schönlein purpura nephritis, ANCA-associated vasculitis, urinary tract infection, autosomal dominant polycystic kidney disease; bacterial infectious diseases, such as sepsis; gestational hypertension; diabetes, gestational diabetes, diabetic nephropathy.

In one or more embodiments, the M1 macrophage-mediated diseases are selected from: hypertrophic scars in regression phase; chronic obstructive pneumonia; tumors, such as breast cancer, liver cancer; metabolic diseases, such as severe obesity, insulin resistance, type II diabetes; inflammatory diseases, such as acute pancreatitis; coronary artery diseases, such as atherosclerosis; nephropathy; obesity; cardiovascular diseases, such as myocarditis, myocardial infarction; cerebral diseases, such as cerebral infarction, cerebral injury; ophthalmic diseases, such as autoimmune uveitis, retinopathy, keratitis, corneal transplantation, sicca syndrome, uveal melanoma; immune inflammatory diseases, such as inflammatory enteritis, autoimmune hepatitis, asthma, alcoholic liver disease, colitis, multiple sclerosis, periodontitis, osteoarthritis; diabetes, gestational diabetes, diabetic nephropathy.

In one or more embodiments, the M2 macrophage-mediated diseases are selected from: hypertrophic scars in proliferative phase; tumors, such as breast cancer, liver cancer; metabolic diseases, such as insulin resistance, type II diabetes; inflammatory diseases, such as acute pancreatitis; cardiovascular diseases, such as myocardial infarction, myocardial failure, atherosclerosis, coronary artery disease, myocarditis; cerebral infarction; ophthalmic diseases, such as autoimmune uveitis, retinopathy, keratitis, corneal transplantation, sicca syndrome, uveal melanoma; immune inflammatory diseases, such as inflammatory enteritis, autoimmune hepatitis, asthma, alcoholic liver disease, colitis, multiple sclerosis, periodontitis, osteoarthritis; diabetes, gestational diabetes, diabetic nephropathy.

In one or more embodiments, the TLR4/NF-κB signaling pathway-mediated diseases are selected from: systemic lupus erythematosus; vascular inflammations, such as atherosclerosis, coronary heart disease; myocarditis, such as myocardial ischemic tissue inflammation, myocardial injury; hepatitis, such as hepatic failure, alcoholic liver injury, inflammatory immune responses during alcohol metabolism; fatty liver; pneumonia, such as acute lung injury, chronic obstructive pulmonary disease, silicosis; nephritis, such as acute kidney injury, lupus nephritis; inflammatory bowel diseases, such as acute enteritis, ulcerative colitis, radiation proctitis; gastritis, such as chronic atrophic gastritis; acute respiratory infections, such as pneumonia, bronchitis, pharyngitis, sinusitis, otitis media; periodontitis, hyperuricemia; rhinallergosis, allergic rhinitis; mastitis; arthritis, such as acute gouty arthritis, chronic arthritis, rheumatoid arthritis; wound tissue inflammation, hypertrophic scars; polycystic ovary syndrome; tumors, such as pituitary prolactinoma, adrenocorticotropic hormone adenoma, intracranial aneurysm; infectious diseases, such as bacterial infections, fungal infections, viral infections; allergic diseases, such as allergic skin diseases, bronchial asthma, allergic rhinitis, Henoch-Schönlein purpura.

### BRIEF DESCRIPTION OF THE DRAWINGS

FIG.1 is a schematic diagram showing ceAF alleviating inflammation in lipopolysaccharide (LPS)-stimulated RAW 264.7 cells via the TLR4/NF-κB signaling pathway. In diagram D, each group of 4 histograms corresponds to CD206, Arg-1, iNOS, TNF-α, IL-6, IL-1β, TLR4, NF-κB, and plκB sequentially from left to right.
FIG.2 is a schematic diagram showing ceAF inducing polarization of RAW264.7 cells toward M2 macrophages *in vitro.*
FIG.3 is a schematic diagram showing ceAF promoting wound healing in STZ-induced diabetic mice.
FIG.4 is a schematic diagram showing ceAF improving histological parameters of wounds in STZ-induced diabetic mice.
FIG.5 is a schematic diagram showing ceAF improving wound healing-related indicators of STZ-induced diabetic mice.
FIG.6 is a schematic diagram showing ceAF regulating wound-related inflammation cytokines in STZ-induced diabetic mice. In diagram B, each group of 2 histograms corresponds to CD206, Arg-1, iNOS, TNF-α, IL-6, and IL-1β sequentially from left to right.

### DETAILED DESCRIPTION

It should be appreciated that the aforementioned technical features and the technical features specifically described below (for example, embodiments) of the present invention can be combined with each other to constitute preferred technical solutions.

The present invention has discovered that chicken embryo amniotic fluid (ceAF) can induce the transformation of M1 macrophages into M2 macrophages via the TLR4/NF-κB signaling pathway, thus completing the present invention.

### Amniotic Fluid

Amniotic fluid can be derived from avian eggs and non-human mammals. Poultry eggs refer to eggs of poultry. Preferred poultry are domestic fowls, such as chickens, ducks and geese. Preferably, poultry eggs with an embryonic age of 5-20 days, preferably 6-15 days are used in the present invention. It should be understood that the appropriate embryonic ages for different eggs may not be the same. For example, when chicken eggs are used, preferably eggs with an embryonic age of 5-12 days, more preferably eggs with an embryonic age of 6-11 days, still more preferably eggs with an embryonic age of 7-9 days, and still more preferably eggs with an embryonic age of 7-8 days are used. When eggs from other poultry species are used, eggs whose development stage corresponds to the development stage of chicken eggs of the embryonic ages may be used. For example, when duck eggs are used, duck eggs with an embryonic age of 8-10 days, particularly 8-9 days, may be best. In some specific embodiments, ceAF refers to amniotic fluid extracted from chicken embryos incubated for 6-8 days.

Amniotic fluid of poultry eggs can be obtained using conventional methods. For example, the blunt end of an egg of corresponding embryonic age can be knocked to break the eggshell, and the eggshell can be peeled off to form a hole about 2 cm in diameter. Then the shell membrane and vitelline membrane are torn off carefully with tweezers, attention should be paid not to damage the amniotic membrane. The amniotic membrane and connected tissues surrounding the embryo are poured from the shell into a petri dish, and a syringe is used to pierce the amniotic membrane to extract amniotic fluid until the amniotic membrane is close to the embryo, thereby obtaining amniotic fluid used in the present invention.

In the present invention, amniotic fluid may also be derived from non-human mammals, especially rodents, for example, from mice. Other non-human mammals may be common domestic animals, such as cattle, sheep, dogs, cats, pigs, etc. In some embodiments, the amniotic fluid is derived from an embryo of a rodent with an embryonic age of 8-14 days, or from an embryo of a non-human mammal whose development stage corresponds to the development stage of the rodent with an embryonic age of 8-14 days. Amniotic fluid can be obtained by conventional methods. For example, surgical scissors are used to cut the abdominal cavity of a mouse whose pregnant period is 8-14 days to carefully remove and cut the uterus, and a syringe is used to pierce the amniotic membrane to extract amniotic fluid until the amniotic membrane is close to the embryo, thereby obtaining amniotic fluid used in the present invention.

It should be understood that when necessary, the amniotic fluid may be centrifuged to separate potential impurities, for example, egg yolk, to obtain pure amniotic fluid as much as possible. The supernatant obtained after centrifugation is the amniotic fluid used in the present invention. It should be understood that all steps to obtain amniotic fluid need to be performed under aseptic conditions; in addition, the "amniotic fluid" shown herein should be "pure" amniotic fluid, i.e., amniotic fluid separated from poultry eggs or non-human mammalian embryos that contains no other components in avian eggs or non-human mammalian embryos and is not contaminated by exogenous substances. Pure amniotic fluid may be stored in a refrigerator below -60°C and thawed before use.

The amniotic fluid described herein can be used as an active ingredient of a medicine for *in vivo* administration to a subject in need, promoting cell growth and tissue repair *in vivo.* For example, a subject in need thereof can be administered an effective amount of amniotic fluid described herein, or a pharmaceutical composition containing the amniotic fluid.

It should be appreciated that, in light of CN 201810911038.2, CN 201810909485.4, CN 201810909193.0, and CN 201910887556.X, the various sources of amniotic fluid described herein possess equivalent or comparable biological activities. When applied within the scope of the present invention, all such amniotic fluid sources are capable of regulating the TLR4/NF-κB signaling pathway, inhibiting M1 macrophages, and increasing M2 macrophages. Consequently, they are effective for treating or preventing diseases that benefit from the regulation via the TLR4/NF-κB signaling pathway, as well as macrophage-mediated diseases.

In the present invention, an animal may be a mammal, especially a human.

### Disease

Toll-like receptors (TLRs) are a family of receptors that mediate innate immunity. In particular, TLR4 can recognize a variety of pathogen-associated molecular patterns (PAMPs) and activate inflammatory cells, namely, it is able to recognize the exogenous ligand lipopolysaccharide (LPS) as well as endogenous ligands expressed during arterial damage. At the same time, it can regulate pathological processes such as cholesterol metabolism, plaque stability, cell apoptosis, inflammation, vascular remodeling, and immune responses. After binding to ligands, TLRs ultimately lead to the activation of NF-κB, regulating the expressions of genes related to inflammation and immunity.

NF-κB plays a role in multiple stages of disease pathogenesis, including inflammatory responses, foam cell formation, vascular smooth muscle cell proliferation, and cell apoptosis. It regulates enzymes involved in early processes such as low-density lipoprotein (LDL) modification and inflammatory lipid formation (for example, cyclooxygenase-2). Activated NF-κB also promotes the transcription of adhesion molecules, pro-inflammatory cytokines, chemokines, and growth factors, all of which play critical roles in the onset and progression of inflammatory diseases.

In the present invention, the TLR4/NF-κB signaling pathway-mediated diseases include but not limited to inflammatory responses caused by neuropathies/vasculopathies, which are selected from systemic lupus erythematosus (Ji Juan. The abnormal activation of TLR4 mediates the senescence of BM-MSCs in the pathogenesis of SLE [D]. Nantong University, 2017.); vascular inflammation and atherosclerosis (Li Hongmei, Wang Xian. Research progress on the correlation between TLR4/MyD88/NF-κB signaling pathway and atherosclerotic cardiovascular diseases [J]. Chinese Journal of Evidence-Based Cardiovascular Medicine, 2017, 9(09): 1132-1134.), coronary heart disease (Lin Yongjun et al. Effect of ginkgo leaf dripping pill on TLR4/NF-κB inflammatory signal pathway and immune function of patients with coronary heart diseases [J]. Strait Pharmaceutical Journal, 2017, 29(03): 89-92.); myocarditis, including myocardial ischemic tissue inflammation (Zhou Xueling. Huoxin Pill against acute myocardial ischemia inflammatory injury through the inhibition of TLR4/NF-κB signaling pathway [D]. Fujian University of Traditional Chinese Medicine, 2020. DOI: 10.27021/d.cnki.gfjzc.2020.000334.), myocardial injury (Wei Hao et al. Protective mechanism of HIF-1α on myocardial injury in rats with myocardial ischemia-reperfusion by TLR4/NF-κB signaling pathway [J]. Journal of Clinical and Experimental Medicine, 2019, 18(10): 1017-1020.); hepatitis, including hepatic failure (Liu Qiaohong, Liu Jiangkai, Li Suling. The curative effect of Jiedu Huayu Tongfu Decoction with SALF&ACLF patients and on TLR4/NF-κB [J]. Chinese Journal of Integrated Traditional and Western Medicine on Liver Diseases, 2018, 28(05): 264-267.), alcoholic liver injury (Zhu Xiaoning, Wang Jing, Zhang Yurong, Yin Yue, Peng Mengyun, Zeng Yong. Qutan Huoxue Prescription up-regulates SOCS1 inhibits TLR4/NF-κB signaling pathway to improve liver injury in nonalcoholic steatohepatitis mice [J]. Modernization of Traditional Chinese Medicine and Materia Medica-World Science and Technology, 2020, 22(12): 4293-4299.), inflammatory immune responses during alcohol metabolism (Yin Xiaolei, Lu Weina, Feng Liying. Role of LPS/TLR4 signaling pathway in nonalcoholic fatty liver disease. World Chinese Journal of Digestology 2013; 21(28): 2957-2962. DOI: 10.11569/wcjd.v21.i28.2957.); fatty liver (Yin Xiaolei, Lu Weina, Feng Liying. Role of LPS/TLR4 signaling pathway in nonalcoholic fatty liver disease. World Chinese Journal of Digestology 2013; 21(28): 2957-2962. DOI: 10.11569/wcjd.v21.i28.2957.); pneumonia, such as acute lung injury (Niu Zequn, Wang Liming, Feng Hui, Sun Jiangli, Pei Honghong, Pan Longfei. Effect of TLR4/NF-κB signaling pathway in acute necrotizing pancreatitis-associated acute lung injury of rats [J]. Modern Digestion & Intervention, 2019, 24(12): 1404-1407.), chronic obstructive pulmonary disease (Chen Xunchun, Li Minglan, Pan Biyun, Wang Yanying, Ding Yipeng. Role of LncRNA RP11-20G6 and TLR4/NF-κB signaling pathway in airway inflammation and remodeling in COPD [J]. Acta Universitatis Medicinalis Anhui, 2022, 57(4): 586-593.), silicosis (Zhu Lili. Research the roles of TLR4 and RAGE mediated inflammatory cytokines in pathogenesis of silicosis [D]. Shanxi Medical University, 2014.); nephritis, including acute kidney injury (Zhan Yun, Zhang Yingjie, Leng Bin. The protective effect of Astragaloside IV on acute vascular endothelial injury induced by LPS via NF-κB p65 signaling pathway [J]. Pharmacology and Clinics of Chinese Materia Medica, 2018, 34(03): 77-80. DOI: 10.13412/j.cnki.zyyl.2018.03.019.), lupus nephritis (Chen Ning. Expression and effect of HMGB1/TLR/NF-κB signal pathway in Murine Lupus Nephritis [D]. Hebei Medical University, 2010.); inflammatory bowel diseases, such asacute enteritis (Li Xuehui et al. Effects of CD11b agonist leukadherin-1 on dextran sulfate sodium-induced acute experimental colitis in mice and the underlying mechanism [J]. Chinese Journal of Microbiology and Immunology, 2019(12): 904-905-906-907-908-909-910.), ulcerative colitis (Lin Xiaoyuan, Liu Jiemin. TLR4/MyD88/NF-κB signaling pathway and ulcerative colitis [J]. Chinese Journal of Gastroenterology, 2013, 18(04): 244-246.), radiation proctitis (Zhu Chaofu, An Baiping, Huang Hongjie, Du Chi, Wu Yongjun, Lan Lan, Li Dan, Lei Dongmei, Li Shijie, Ao Rui. Mechanism of modified Tuoli Xiaodu powder in the treatment of radiation proctitis based on TLR4/NF-κB signaling pathway [J]. Acta Universitatis Medicinalis Anhui, 2020, 55(09): 1367-1373. DOI: 10.19405/j.cnki.issn1000-1492.2020.09.010.); gastritis, such as chronic atrophic gastritis (Zhou Wei, Yuan Xingxing. Effect of Periplaneta americana extract on TLR4/NF-κB signaling pathway in gastric mucosa of rats with chronic atrophic gastritis [J]. World Chinese Journal of Digestology, 2017, 25(21): 1945-1951.); acute respiratory infections,such as pneumonia (Guo Shasha. The role of TLR-MYD88-NF-κB P65 signaling pathways in mycoplasma pheumonia lung tissue of the mice [D]. Qingdao University, 2017.), bronchitis (Wu Ning, Huang Yuxiao, Shi Xue, Chen Jinlun, Peng Lingfeng, Yang Lulu, Xu Hong, Sun Jianfei, Liu Hua. Effect of Lysionotus pauciflorus Maxim. on Signaling Pathway of TLR4-MyD88-NF-κB in Lung Tissue of Rats with Chronic Bronchitis [J]. Journal of Guizhou Medical University, 2020, 45(11): 1283-1288. DOI: 10.19367/j.cnki.2096-8388.2020.11.009.), pharyngitis (Xu Jinhong. To investigate the mechanism of treating acute pharyngitis with Zizheng Dihuang decoction based on TLR4/NF-κB signaling pathway [D]. Anhui University of Chinese Medicine, 2020. DOI: 10.26922/d.cnki.ganzc.2020.000216.), sinusitis (Xiao Jianing, Xue Shanshan, Ni Pingmin, Wang Zhuo, Wu Yongjun. Study on the mechanism of Biyuan Heji in the treatment of acute sinusitis based on NF-xB signal pathway [J]. Journal of Nanjing University of Tradtional Chinese Medicine, 2022, 38(3): 247-253. doi: 10.14148/j.issn.1672-0482.2022.0247), otitis media (CN201180025442.6, Luo Luncai, Tong Yan, Zhang Xingguo, et al. Study on the effects of Yuyang Capsules on otitis media in rats based on TLR4/MyD88/NF-κB pathway [J]. Northwest Pharmaceutical Journal, 2021, 36(01): 66-71.); periodontitis, hyperuricemia (Guo Zhuling, Tang Han, Huang Miao, et al. Exploration on the mechanism of interaction between periodontitis and hyperuricemia based on TLR4/NF-κB signaling pathway [J]. Chinese Journal of Stomatological Research (Electronic Edition), 2021, 15(1): 5.); asthma (Li Hongjia. The experimental study on the mechanism of quercetin in asthmatic airway inflammation through TLR4/NF-κB signaling pathway [D]. Shandong University, 2015.); rhinallergosis (Yin Lili. Expression and role of TREM-1, TLR4, TNF-α and NF-κB in nasal mucosa of allergic rhinitis mouse [D]. Huazhong University of Science and Technology, 2013.), allergic rhinitis (Hu Chen, Liang Chenyang, Zhou Weiguo. Expression and role of TLR4/NF-κB pathway in patients with allergic rhinitis [J]. Labeled Immunoassays and Clinical Medicine, 2018, 25(06): 788-790+838.); mastitis (Lu Jinye, Gu Beibei. Progress in research on the correlation between the TLR4/MyD88/NF-κB signaling pathway and mastitis [J]. Animal Husbandry & Veterinary Medicine, 2018, 50(11): 127-129.); arthritis, such as acute gouty arthritis (LUO Fei, MEI Yan. Effects of procyanidins on TLR4/NF-κB signaling pathway in rats with acute gouty arthritis. Chinese Journal of Clinical Pharmacology and Therapeutics. 2018, (1): 41-46. DOI: 10.12092/j.issn.1009-2501.2018.01.008), chronic arthritis (Yu Bijun. The role of Toll-like receptor signal transduction pathway in the chronic inflammatory arthritis [J]. International Journal of Immunology, 2007, 30(06): 443-447.), rheumatoid arthritis (Bai Lin, Yang Yuxin, Wan Qiaofeng, et al. Baicalin alleviated rheumatoid arthritis synovitis of SD rats through TLR2-NF-κB pathway [J]. Chinese Pharmacological Bulletin, 2017, 33(11): 1569-73.); wound tissue inflammation, hypertrophic scars (Li Qiang. Expression and significance of TRAIL and DR5 and NF-κB in human chronic granulation tissue and hypertrophic scar [D]. Shandong University, 2008.); polycystic ovary syndrome (Yao Zhilin, Huang Yinghong, Xu Xiaojuan. Effect of Bushen Huayu Recipe combined with metformin on TLR4/NF-κB inflammatory signal pathway, oxidative stress and insulin resistance in female rats with polycystic ovary syndrome [J]. Journal of Sichuan Traditional Chinese Medicine, 2019, 37(11): 6.); pituitary prolactinoma (Guo Runzhu. Study on the pharmacodynamic effect and mechanism of hordenine on hyperprolactinemia and prolactinoma [D]. Hubei University of Chinese Medicine, 2019. DOI: 10.27134/d.cnki.ghbzc.2019.000015.), adrenocorticotropic hormone adenoma (Wu Q, Feng Y, Liu L, Liu Y, Liu X, Zhang L, Li Y, Wang L. Corticotropin-Releasing Factor Aggravates Ischemic Stroke Injury by the Inflammatory Activation of Microglia. Endocrinology. 2022 Mar 1; 163(3):bqac013. doi: 10.1210/endocr/bqac013. PMID: 35137012.), intracranial aneurysm (Wang Y, Jin J. Roles of macrophages in formation and progression of intracranial aneurysms. Zhejiang Da Xue Xue Bao Yi Xue Ban. 2019 Apr 25; 48(2): 204-213. Chinese. doi: 10.3785/j.issn.1008-9292.2019.04.13. PMID: 31309760; PMCID: PMC8800668.) and other tumors' occurrence and progression; infectious diseases, such as bacterial infections, fungal infections and viral infections (Shan Jialing, Cheng Hongyu, Wen Le, Zhong Guoyue, Zhu Jixiao. Advances in research of TLR/MyD88/NF-κB signaling pathway in different diseases [J]. Chinese Pharmacological Bulletin, 2019, 35(4): 451-455); allergic diseases, such as allergic skin diseases, bronchial asthma, allergic rhinitis (Shan Jialing, Cheng Hongyu, Wen Le, Zhong Guoyue, Zhu Jixiao. Advances in research of TLR/MyD88/NF-κB signaling pathway in different diseases [J]. Chinese Pharmacological Bulletin, 2019, 35(4): 451-455) and Henoch-Schönlein purpura (Wang Ziwei, Yang Lijun. Expression and significance of TLR9, MyD88 and NF-κB in HSP [J]. China Modern Doctor, 2016, 54(29): 9-12).

The amniotic fluid described herein can inhibit the activation of the TLR4/NF-κB signaling pathway, thereby treating or preventing various TLR4/NF-κB signaling pathway-mediated diseases.

In the present invention, macrophage mediation refers to the roles of dynamic balance and imbalance among macrophage subpopulations with different functional characteristics in the pathological process of diseases. Macrophage-mediated diseases include but are not limited to hypertrophic scars (Li Zhenjiang, Li Shujun, Zhou Jian, et al. The study on macrophage activation related factors in hypertrophic scar tissue at different periods [J]. Journal of Zunyi Medical University, 2022, 45(1): 87-91); chronic obstructive pneumonia (Xing Shigang. Research on the role of macrophages in the pathogenesis of chronic obstructive pulmonary disease [J]. China Practical Medicine, 2019, 14(12): 196-197); tumors, such as breast cancer, liver cancer; metabolic diseases, such as severe obesity, insulin resistance, type II diabetes; inflammatory diseases, such as acute pancreatitis, atherosclerosis (Wang Yongkang, Li Jiayi, Guan Fei, Lei Jiahui. Mechanism of macrophage polarization and its role in common diseases [J]. Journal of Tropical Diseases and Parasitology, 2022, 20(2): 103-108, 112); cardiovascular diseases, such as myocarditis, myocardial infarction, cardiac arrhythmia (Dong Jingwei, Miao Liu. Advances in macrophage functions and its role in heart disease [J]. China Modern Medicine, 2022, 29(16): 49-52); neuropathies, such as Alzheimer's disease; cerebral diseases, such as cerebral infarction (Wang Jiahe. Preface - Research progress on macrophages and related diseases [J]. Practical Geriatrics, 2021, 35(12): 1217-1218), cerebral injury (Diao Zhongji, Lei Rui, Yin Shi, Liu Hongling, Liu Qing. Correlation between M1 macrophage level and severity and prognosis of coronary heart disease [J]. Medical Innovation of China, 2022, 19(4): 161-165); ophthalmic diseases, such as autoimmune uveitis, retinopathy, keratitis, corneal transplantation, sicca syndrome, uveal melanoma, myopia (Qu Ruyi, Zhou Mengxian, Bi Hongsheng, Guo Dadong. Research advances in the role of macrophage polarization in the occurrence and development of ophthalmic diseases [J]. Recent Advances in Ophthalmology, 2022, 42(3): 239-243); immune inflammatory diseases, such as inflammatory enteritis, autoimmune hepatitis, asthma (Jia Rui, Hui Yi, Yan Shuguang (supervisor), Li Jingtao. Research progress on relationship between macrophage M1/ M2 polarization and immune inflammatory diseases [J]. Chinese Journal of Immunology, 2021, 37(22): 2791-2797), alcoholic liver disease, colitis (Wu Yan, Zhang Dingran, Wang Xinhui, Xu Hongyang, Liu Peiyao, Qi Zhili. Advances in macrophage polarization and its effects on inflammatory diseases [J]. Chinese Journal of Animal Science, 2021, 57(7): 22-26), multiple sclerosis (Li Xing, Wang Dandan, Tang Qi, Liu Jie, Gu Zhongyi, Zhao Huan, Sun Hongchen. Advanced research on transcription regulation of macrophage polarization and its influences in related diseases [J]. Journal of Jilin University (Medicine Edition), 2016, 0(3): 622-625), periodontitis (Zhou Qi, Sun Huijuan, Yu Donghua, Liu Shumin. Mechanism of M1/M2 macrophage polarization in different diseases [J]. Chinese Pharmacological Bulletin, 2020, 36(11): 1502-1506); arthritis, such as rheumatoid arthritis, bone erosion, synovitis (Wang Dongyi, Shen Junyi, Lu Le, Cai Hui. Association of macrophage polarization imbalance with disease activity and bone erosion in rheumatoid arthritis [J]. Journal of Medical Postgraduates, 2021, 34(8): 823-828), osteoarthritis (Zhou Qi, Sun Huijuan, Yu Donghua, Liu Shumin. Mechanism of M1/M2 macrophage polarization in different diseases [J]. Chinese Pharmacological Bulletin, 2020, 36(11): 1502-1506); nephritis, such as acute kidney injury, chronic kidney disease, end-stage renal disease, proliferative glomerulonephritis, membranous nephropathy, diabetic nephropathy, Henoch-Schönlein purpura nephritis, ANCA-associated vasculitis, urinary tract infection, autosomal dominant polycystic kidney disease (Ding Na, Wang Bo, Huang Bintao, Hao Jian. Research progress on macrophage migration inhibitory factor in the pathogenesis of nephritic diseases [J]. Hebei Medicine, 2021, 27(1): 170-174); bacterial infectious diseases, such as sepsis (Huang Xuechao, Shen Shiyang, Mo Ran. Advances in macrophage membrane-based biomimic nano-drug delivery system for inflammatory disease treatment [J]. Chinese Journal of Drug Evaluation, 2021, 38(4): 279-283); gestational hypertension (Zhao Caizhen, Qiao Fuyuan. Macrophages and gestational hypertension [J]. Chinese Journal of Birth Health & Heredity, 2006, 14(3): 126-128); diabetes, gestational diabetes, diabetic nephropathy (Zhou Qi, Sun Huijuan, Yu Donghua, Liu Shumin. Mechanism of M1/M2 macrophage polarization in different diseases [J]. Chinese Pharmacological Bulletin, 2020, 36(11): 1502-1506).

Macrophages comprise two major subtypes, i.e., M1 and M2. M1 macrophages, also known as pro-inflammatory phenotype macrophages, primarily function in the body to phagocytose bacteria and other foreign substances as well as endogenous materials such as apoptotic cell debris, thereby protecting tissues and organs from invasion by foreign substances. In inflammatory diseases, M1 macrophages accumulate at early inflammatory sites and are activated by pro-inflammatory cytokines. They promote the occurrence and progression of inflammation through the secretion of inflammatory cytokines and protect the body from invasion by foreign substances. In the late stage of inflammation, M2 macrophages play roles in inhibiting inflammation, repairing tissues, and reconstructing tissue structures. Therefore, M2 macrophages are also known as anti-inflammatory phenotypes or immunoregulatory macrophages. During the occurrence and progression of inflammation, the ratio between M1 and M2 macrophage populations continuously changes over time, eventually eliminating the effects of inflammation completely.

In the present invention, M1 macrophage mediation refers to the roles of M1 macrophages in diseases, including being activated by pro-inflammatory cytokines, secreting inflammatory cytokines, and protecting the body from invasion by foreign substances. M1 macrophage-mediated diseases include but are not limited to hypertrophic scars in regression phase (Li Zhenjiang, Li Shujun, Zhou Jian, et al. The study on macrophage activation related factors in hypertrophic scar tissue at different periods [J]. Journal of Zunyi Medical University, 2022, 45(1): 87-91); chronic obstructive pneumonia (Xing Shigang. Research on the role of macrophages in the pathogenesis of chronic obstructive pulmonary disease [J]. China Practical Medicine, 2019, 14(12):196-197); tumors, such as breast cancer, liver cancer; metabolic diseases, such as severe obesity, insulin resistance, type II diabetes; inflammatory diseases, such as acute pancreatitis (Wang Yongkang, Li Jiayi, Guan Fei, Lei Jiahui. Mechanism of macrophage polarization and its role in common diseases [J]. Journal of Tropical Diseases and Parasitology, 2022, 20(2): 103-108, 112); coronary artery diseases, such as atherosclerosis; nephropathy; obesity (Diao Zhongji, Lei Rui, Yin Shi, Liu Hongling, Liu Qing. Correlation between M1 macrophage level and severity and prognosis of coronary heart disease [J]. Medical Innovation of China, 2022, 19(4): 161-165); cardiovascular diseases, such as myocarditis, myocardial infarction (Dong Jingwei, Miao Liu. Advances in macrophage functions and its role in heart disease [J]. China Modern Medicine. 2022, 29(16): 49-52); cerebral diseases, such as cerebral infarction (Wang Jiahe. Preface - Research progress on macrophages and related diseases [J]. Practical Geriatrics, 2021, 35(12): 1217-1218), cerebral injury (Diao Zhongji, Lei Rui, Yin Shi, Liu Hongling, Liu Qing. Correlation between M1 macrophage level and severity and prognosis of coronary heart disease [J]. Medical Innovation of China, 2022, 19(4): 161-165); ophthalmic diseases, such as autoimmune uveitis, retinopathy, keratitis, corneal transplantation, sicca syndrome, uveal melanoma (Qu Ruyi, Zhou Mengxian, Bi Hongsheng, Guo Dadong. Research advances in the role of macrophage polarization in the occurrence and development of ophthalmic diseases [J]. Recent Advances in Ophthalmology, 2022, 42(3): 239-243); immune inflammatory diseases, such as inflammatory enteritis, autoimmune hepatitis, asthma (Jia Rui, Hui Yi, Yan Shuguang (supervisor), Li Jingtao. Research progress on relationship between macrophage M1/ M2 polarization and immune inflammatory diseases [J]. Chinese Journal of Immunology, 2021, 37(22): 2791-2797), alcoholic liver disease, colitis (Wu Yan, Zhang Dingran, Wang Xinhui, Xu Hongyang, Liu Peiyao, Qi Zhili. Advances in macrophage polarization and its effects on inflammatory diseases [J]. Chinese Journal of Animal Science, 2021, 57(7): 22-26), multiple sclerosis (Li Xing, Wang Dandan, Tang Qi, Liu Jie, Gu Zhongyi, Zhao Huan, Sun Hongchen. Advanced research on transcription regulation of macrophage polarization and its influences in related diseases [J]. Journal of Jilin University (Medicine Edition), 2016, 0(3): 622-625), periodontitis, osteoarthritis; diabetes, gestational diabetes, diabetic nephropathy (Zhou Qi, Sun Huijuan, Yu Donghua, Liu Shumin. Mechanism of M1/M2 macrophage polarization in different diseases [J]. Chinese Pharmacological Bulletin, 2020, 36(11): 1502-1506).

In some embodiments, M1 macrophage-mediated diseases include but are not limited to immune inflammatory diseases, metabolic diseases, diabetes, and tumors. In an exemplary embodiment, the M1 macrophage-mediated disease described herein is persistent chronic inflammation in diabetic wounds.

In the present invention, M2 macrophage mediation refers to the roles of M2 macrophages in disease contexts, including suppressing inflammation and inflammatory cytokines, and promoting wound healing and tissue repair. M2 macrophage-mediated diseases include but are not limited to hypertrophic scars in proliferative phase (Li Zhenjiang, Li Shujun, Zhou Jian, et al. The study on macrophage activation related factors in hypertrophic scar tissue at different periods [J]. Journal of Zunyi Medical University, 2022, 45(1): 87-91); tumors, such as breast cancer, liver cancer; metabolic diseases, such as insulin resistance, type II diabetes; inflammatory diseases, such as acute pancreatitis (Wang Yongkang, Li Jiayi, Guan Fei, Lei Jiahui. Mechanism of macrophage polarization and its role in common diseases [J]. Journal of Tropical Diseases and Parasitology, 2022, 20(2): 103-108, 112); cardiovascular diseases, such as myocardial infarction, myocardial failure, atherosclerosis, coronary artery disease (Zhang Xiangning, Dang Guohui, Feng Juan (supervisor). Exosome-mediated regulation of M2 macrophages and its role in cardiovascular diseases [J]. Chinese Journal of Immunology, 2022, 38(10): 1257-1262), myocarditis (Dong Jingwei, Miao Liu. Advances in macrophage functions and its role in heart disease [J]. China Modern Medicine, 2022, 29(16): 49-52); cerebral infarction (Wang Jiahe. Preface - Research progress on macrophages and related diseases [J]. Practical Geriatrics, 2021, 35(12): 1217-1218); ophthalmic diseases, such as autoimmune uveitis, retinopathy, keratitis, corneal transplantation, sicca syndrome, uveal melanoma (Qu Ruyi, Zhou Mengxian, Bi Hongsheng, Guo Dadong. Research advances in the role of macrophage polarization in the occurrence and development of ophthalmic diseases [J]. Recent Advances in Ophthalmology, 2022, 42(3): 239-243); immune inflammatory diseases, such as inflammatory enteritis, autoimmune hepatitis, asthma (Jia Rui, Hui Yi, Yan Shuguang (supervisor), Li Jingtao. Research progress on relationship between macrophage M1/ M2 polarization and immune inflammatory diseases [J]. Chinese Journal of Immunology, 2021, 37(22): 2791-2797), alcoholic liver disease, colitis (Wu Yan, Zhang Dingran, Wang Xinhui, Xu Hongyang, Liu Peiyao, Qi Zhili. Advances in macrophage polarization and its effects on inflammatory diseases [J]. Chinese Journal of Animal Science, 2021, 57(7): 22-26), multiple sclerosis (Li Xing, Wang Dandan, Tang Qi, Liu Jie, Gu Zhongyi, Zhao Huan, Sun Hongchen. Advanced research on transcription regulation of macrophage polarization and its influences in related diseases [J]. Journal of Jilin University (Medicine Edition), 2016, 0(3): 622-625), periodontitis, osteoarthritis; diabetes, gestational diabetes, diabetic nephropathy (Zhou Qi, Sun Huijuan, Yu Donghua, Liu Shumin. Mechanism of M1/M2 macrophage polarization in different diseases [J]. Chinese Pharmacological Bulletin, 2020, 36(11): 1502-1506).

In some embodiments, M2 macrophage-mediated diseases include but are not limited to immune inflammatory diseases, metabolic diseases, diabetes, and tumors. In an exemplary embodiment, the M2 macrophage-mediated disease described herein is persistent chronic inflammation of diabetic wounds.

In some embodiments, the amniotic fluid described herein is used for the treatment and prevention of macrophage-mediated diseases by reducing the number of M1 macrophages and increasing the proportion of M2 macrophages.

In particularly preferred embodiments of the present invention, amniotic fluid, especially the amniotic fluid from poultry eggs described herein, more preferably the amniotic fluid from chicken eggs is used for the prevention or treatment of macrophage-mediated diseases.

### Pharmaceutical composition

The present invention also provides a pharmaceutical composition, which contains the amniotic fluid described herein, especially the amniotic fluid from poultry eggs, more preferably the amniotic fluid form chicken eggs with an embryonic age of 5-12 days, still more preferably 6-11 days, still more preferably 6-9 days and still more preferably 7-8 days. The pharmaceutical composition may be amniotic fluid or lyophilized reagent thereof frozen and stored below -60°C, for example, lyophilized amniotic fluid. The pharmaceutical composition may also contain other pharmaceutically acceptable carriers or excipients, for example, physiological saline for injection, water for injection, or glucose injection, etc. Preferably, the pharmaceutical composition contains 5-40% (v/v) or 10%-35%, preferably 15-30% of amniotic fluid.

In general, a pharmaceutical composition containing the amniotic fluid also contains pharmaceutically acceptable excipients. In the present invention, "pharmaceutically acceptable excipients" refer to carriers, diluents and/or excipients that are pharmacologically and/or physiologically compatible with a subject and an active ingredient, including but not limited to antibiotics, humectants, pH regulators, surfactants, carbohydrates, adjuvants, antioxidants, chelating agents, ionic strength enhancers, preservatives, carriers, flow aids, sweeteners, dyes/colorants, flavoring agents, wetting agents, dispersing agents, suspending agents, stabilizers, isotonic agents, solvents or emulsifiers. In some embodiments, pharmaceutically acceptable excipients may include one or more inactive ingredients, including but not limited to: stabilizers, preservatives, additives, adjuvants, sprays, compressed air or other appropriate gases, or other appropriate inactive ingredients used in combination with pharmacologically active compounds. More specifically, appropriate pharmaceutically acceptable excipients may include those commonly used in the treatment of diabetic foot ulcers in the art. In one or more embodiments, appropriate pharmaceutically acceptable excipients for use in sprays are selected from one or more of the following: water, gluconolactone, sodium benzoate, arbutin, sodium hyaluronate, niacinamide, and glycerin. In one or more embodiments, appropriate pharmaceutically acceptable excipients for use in topical agents or dressings are selected from one or more of the following: water, glycerin, panthenol, magnesium ascorbyl phosphate, niacinamide, sodium hyaluronate, phenoxyethanol, caprylyl glycol, and sorbic acid. The content of pharmaceutically acceptable excipients can be determined according to actual conditions in the art.

It is necessary to add dressings when a medicine is sprayed, spread, or applied to wounds. "Dressing" refers to materials used for bandaging wounds, in order to cover ulcers, wounds, or other injuries. Types of wound dressings include passive dressings, interactive dressings, and bioactive dressings. Wound dressings appliable to the uses hereof are understood in the art.

In addition to amniotic fluid, the pharmaceutical composition disclosed herein may further comprise other active ingredients that facilitate a patient's wound healing. These ingredients may include but are not limited to *Centella asiatica* extract, rose hydrosol, licorice root extract, olive leaf extract, calendula flower extract, white willow bark extract, lavender extract, lemon fruit extract, hydrolyzed soy protein, Chinese mugwort leaf extract, tea leaf extract, thyme extract, purple coneflower extract, *Hypericum perforatum* flower/leaf extract, *Aloe barbadensis* leaf juice powder, and yeast extract.

### Uses and therapeutic methods

The present invention further provides use of amniotic fluid described in any of the embodiments herein in the preparation of formulations for treating and/or preventing TLR4/NF-κB signaling pathway-mediated diseases. In another aspect, the present invention provides use of amniotic fluid described in any of the embodiments herein in the preparation of formulations for treating and/or preventing macrophage-mediated diseases. In another aspect, the present invention provides use of amniotic fluid described in any of the embodiments herein in the preparation of formulations for treating and/or preventing M1 macrophage-mediated diseases. In another aspect, the present invention provides use of amniotic fluid described in any of the embodiments herein in the preparation of formulations for treating and/or preventing M2 macrophage-mediated diseases. In another aspect, the present invention provides use of amniotic fluid described in any of the embodiments herein in the preparation of reagents for inhibiting the number of M1 macrophage populations and increasing the proportion of M2 macrophage types. In yet another aspect, the present invention provides use of amniotic fluid described in any of the embodiments herein in the preparation of formulations that promote the transformation of M1 macrophages into M2 macrophages.

In certain embodiments, the present invention provides use of amniotic fluid described in any of the embodiments herein in the preparation of reagents for one or more of the following uses: (1) accelerating a patient's wound healing; (2) promoting neovascularization at the wound site of a patient; (3) reducing the levels of pro-inflammatory cytokines IL-6 and TNF-α and increasing the levels of TGF-β1 and IL-10 of a patient under inflammatory conditions; (4) promoting the formation of type III collagen at a patient's wound site, or increasing the ratio of type III to type I collagen at a patient's wound site; and (5) downregulating the transcription and translation levels of iNOS, TNF-α, IL-6, and IL-1β of a patient under inflammatory conditions, while upregulating the transcription and translation levels of CD206 and Arg-1 of the patient.

In some embodiments, the present invention also provides use of amniotic fluid or a pharmaceutical composition containing the amniotic fluid in any of the embodiments described herein for treating and/or preventing TLR4/NF-κB signaling pathway-mediated diseases, for treating and/or preventing macrophage-mediated diseases, for treating and/or preventing M1 macrophage-mediated diseases, for treating and/or preventing M2 macrophage-mediated diseases, for inhibiting the number of M1 macrophage populations and increasing the proportion of M2 macrophage types, and/or for promoting the transformation of M1 macrophages into M2 macrophages.

The present invention further provides a method for treating and/or preventing TLR4/NF-κB signaling pathway-mediated diseases, comprising a step of administering an effective amount of the amniotic fluid of the present invention or a pharmaceutical composition containing the amniotic fluid to a subject in need.

The present invention also provides a method for treating and/or preventing macrophage-mediated diseases. The present invention also provides a method for treating and/or preventing M1 macrophage-mediated diseases. The present invention also provides a method for treating and/or preventing M2 macrophage-mediated diseases. These methods comprise a step of administering an effective amount of the amniotic fluid of the present invention or a pharmaceutical composition containing the amniotic fluid to a subject in need.

The present invention further provides a method for promoting the transformation of M1 macrophages into M2 macrophages, comprising a step of administering an effective amount of the amniotic fluid of the present invention or a pharmaceutical composition containing the amniotic fluid to a subject in need. In some embodiments, the method can be used for treating or preventing diseases that benefit from increased M2 macrophages, including but not limited to the aforementioned diseases. The method may be implemented *in vivo* or *in vitro.*

The present invention also provides a method for repairing tissues of a subject under inflammatory conditions. The method comprises a step of using amniotic fluid described herein or a composite dressing prepared with the amniotic fluid as the main ingredient, or culturing tissue cells of interest *in vitro* using a cell culture medium containing the amniotic fluid described herein, and upon the formation of a tissue matrix, implanting the tissue matrix into the tissue injury or defect site.

In some embodiments, the present invention also provides a method for accelerating a patient's wound healing. The method comprises a step of topically applying the amniotic fluid described herein or a pharmaceutical composition containing the amniotic fluid to the patient's wound, or externally applying a composite dressing formulated with the amniotic fluid of the present invention as the main ingredient. In some embodiments, the present invention also provides a method for promoting neovascularization at the wound site of a patient. The method comprises a step of topically applying the amniotic fluid described herein or a pharmaceutical composition containing the amniotic fluid to the patient's wound, or externally applying a composite dressing formulated with the amniotic fluid of the present invention as the main ingredient. In some embodiments, the present invention also provides a method for reducing the levels of pro-inflammatory cytokines IL-6 and TNF-α and increasing the levels of TGF-β1 and IL-10 of a patient under inflammatory conditions. The method comprises a step of administering an effective amount of the amniotic fluid of the present invention or a pharmaceutical composition containing the amniotic fluid to the patient. In some embodiments, the present invention also provides a method for promoting type III collagen formation at a patient's wound site, or a method for increasing the ratio of type III collagen to type I collagen at the patient's wound site. The method comprises a step of topically applying the amniotic fluid described herein or a pharmaceutical composition containing the amniotic fluid to the patient's wound, or externally applying a composite dressing formulated with the amniotic fluid herein invention as the main ingredient. In some embodiments, the present invention also provides a method for downregulating the transcription and translation levels of iNOS, TNF-α, IL-6, and IL-1β of a patient under inflammatory conditions, while upregulating the transcription and translation levels of CD206 and Arg-1 of the patient. The method comprises a step of administering an effective amount of the amniotic fluid of the present invention or a pharmaceutical composition containing the amniotic fluid to the patient.

In the present invention, an effective amount refers to a dose administered to a subject to achieve the treatment, prevention, alleviation and/or relief of a disease or a disorder. A therapeutically effective dose can be determined according to the factors such as a patient's age, sex, disorder and severity, and other physical conditions of the patient. In the present invention, a subject or a patient generally refers to a mammal, especially a human.

In the present invention, the dosage and frequency of administration can be determined by medical staff according to the patient's specific condition, age and sex, etc. Generally, for the treatment of a particular disease, a therapeutically effective dose refers to an amount sufficient to ameliorate or alleviate the symptoms associated with the disease in a manner. Such an amount may be administered as a single dose or administered according to an effective treatment regimen. Doses may be administered to cure a disease, but are usually administered to ameliorate the symptoms of a disease. Repeated dosing is generally required to achieve the desired improvement of symptoms. For example, the dosage for human can usually be 1-200 ml/time, and can be administered by injection daily or weekly. In some embodiments, the frequency of administration may be multiple times per day, twice per day, once every two days, every three days, every four days, every five days, or every six days, or once every half a month or once a month.

### Cell culture medium

In some embodiments, the present invention also provides a cell culture medium containing an appropriate amount of the amniotic fluid described herein. The content of amniotic fluid in cell culture medium can be determined according to the type of cells being cultured. For example, the amount of amniotic fluid added can be 0.1-30% of the weight of the cell culture medium, such as 1-25% or 3-20%. An appropriate cell culture medium may be selected according to the specific cells to be cultured. Exemplary cell culture media include but are not limited to various commercially available media such as DMEM, RPMI 1640, MEM, and DMEM/F12.

The present invention will be further illustrated below in combination with specific embodiments. It should be understood that these embodiments are illustrative only and are not intended to limit the scope of the present invention. Unless otherwise stated, methods and reagents used in the embodiments are conventional methods and reagents in the art.

### Materials and methods

### Antibodies and reagents

PE-conjugated CD206 antibody (12-2069-42) and FITC-conjugated F4/80 antibody (11-4801-82) were purchased from eBioscience. Arginase 1 (Arg-1; 93668), α-SMA (19245), and CD206 (24595) were purchased from Cell Signaling Technology (CST). GAPDH (ab181602), iNOS (ab178945), TNF-α (ab183218), IL-6 (ab290735), IL-1β (ab254360), and CD31 (ab281583) were purchased from Abcam. TLR4 (A5258), NF-κB-p65 (A19653), and phosphorylated IκB (p-IκB; AP0707) were purchased from ABclonal. Trizol Reagent and SYBR Green were purchased from Vazyme Biotech. Streptozotocin (STZ; S0130) and glucose (D9434) were purchased from Sigma-Aldrich.

### Preparation of ceAF

Fertilized eggs were incubated at 38°C and 50% relative humidity. ceAF was separated at 6 to 8 days of incubation. After centrifugation (2500×g, 20 min), the supernatant was filtered with a 0.22 µm sterile filter (Millipore, USA). The filtered samples were aliquoted and stored at -80°C.

### Cell culture

RAW264.7 cells were provided by the National Collection of Authenticated Cell Cultures (NCACC). Cells were cultured in DMEM high-glucose medium containing 10% FBS and 1% DAB at 37°C and 5% CO₂. The high-glucose condition was the DMEM containing 40 mM glucose. Different concentrations (0%, 1%, 5%, 10%, and 20%) of ceAF were added to the medium for subsequent experiments.

### Animals and wound management

The experimental protocol was approved by the Animal Protection and Ethics Committee of Nanjing Drum Tower Hospital. C57BL/6 mice (male, 8 weeks old) were purchased from the Model Animal Research Center of Nanjing University. The mice were placed in a specific pathogen-free (SPF) environment with unlimited access to water and food. Each group of 18 mice received a daily intraperitoneal injection of 50 mg/kg streptozotocin (STZ, dissolved in sodium citrate buffer) for 5 consecutive days to establish an STZ-induced diabetes model. Three weeks later, the blood glucose levels of mice were measured and those at levels exceeding 16.7 mM were classified as diabetic mice. To establish an excision wound model, 8 mm circular biopsy perforations were performed in the depilated dorsal skin areas of mice. After modeling, 10% ceAF was topically applied to the wound every day, and the control mice were given isometric PBS. Wound images were shot on Day 0, Day 3, Day 5, Day 7, and Day 11, and the wound area was measured using ImageJ (National Institutes of Health). Wound tissue samples were collected on Day 5 and Day 10 after being damaged for subsequent experiments.

### Histological and immunofluorescence staining

Wound margin tissues were fixed, dehydrated, embedded in paraffin, and then sectioned at a thickness of 5 µm. Masson's trichrome (MT), hematoxylin-eosin (H&E), and sirius red staining were performed according to standardized histological procedures. To evaluate macrophage polarization and angiogenesis, CD206, iNOS, CD31, and α-SMA monoclonal antibody (1 µg/ml) were stained overnight at 4°C. Then specific fluorescent secondary antibodies were incubated, followed by DAPI staining.

RAW264.7 cells were sequentially rinsed with PBS, fixed in paraformaldehyde (4%), perforated with 0.1% Triton X-100, and blocked using BSA (3%). Then cells were incubated with the respective primary and secondary antibodies according to the instructions. All images were shot using an Olympus FluoView FV 3000 confocal microscope (Tokyo, Japan).

### RNA isolation and RT-qPCR

Cells and wound margin tissues were treated with Trizol to isolate the total RNA according to the manufacturer's instructions. RT-qPCR was conducted by SYBR Green using the StepOne RT-qPCR system (Applied Biosystems, USA). After normalization with GAPDH, relative gene levels were determined using the 2^{-ΔΔCT} method. The primer sequences are shown in Table 1.

**Table 1: RT-qPCR prime sequences (SEQ ID NO: 1-14)**

| Name | Forward primer | Reverse primer |
|---|---|---|
| *CD206* | GAGGGAAGCGAGAGATTATGGA | GCCTGATGCCAGGTTAAAGCA |
| *Arg-1* | TTGGGTGGATGCTCACACTG | GTACACGATGTCTTTGGCAGA |
| *iNOS* | ACATCGACCCGTCCACAGTAT | CAGAGGGGTAGGCTTGTCTC |
| *TNF-a* | CTGAACTTCGGGGTGATCGG | GGCTTGTCACTCGAATTTTGAGA |
| *IL-6* | CCAAGAGGTGAGTGCTTCCC | CTGTTGTTCAGACTCTCTCCCT |
| *IL-1β* | GAAATGCCACCTTTTGACAGTG | TGGATGCTCTCATCAGGACAG |
| *GAPDH* | CCAGTATGACTCCACTCACG | GACTCCACGACATACTCAGC |

### Western blotting (WB) analysis

Protein samples were separated from lysed skin tissues and cells using RIPA Lysis Buffer (KeyGEN, China). The BCA assay was performed to determine the total protein concentration after centrifugation. Protein samples were separated by 10% SDS-PAGE and transferred to PDVF membranes (Millipore, USA). After blocking with 5% BSA, the membranes were incubated with the corresponding primary antibodies overnight and the secondary antibodies for 1 h. Protein bands were visualized using an ECL Substrate Kit (Vazyme, China).

### Flow cytometry

To determine the polarization trend of RAW264.7 macrophages, cells were preincubated with FITC-conjugated anti-mouse F4/80 antibody and PE-conjugated anti-mouse CD206 antibody for 30 min at 4°C under the condition away from light. Cell phenotype was determined using a flow cytometer (FACSCanto II, BD, USA) and data analysis was performed using FlowJo.

### Cell viability test

The viability of RAW264.7 cells was evaluated using the CCK-8 assay (Beyotime, China). After starvation for 12 h, cells were exposed to 0%, 1%, 5%, 10%, or 20% ceAF, followed by incubation for 24 hours. The cells were rinsed three times with PBS, then covered with 200 µL of incomplete medium (10 µL) containing CCK-8 mixture and incubated at 37°C. The optical density (OD) was measured at 450 nm by an ELISA analyzer.

### Enzyme linked immunosorbent assay (ELISA)

RAW264.7 cells were exposed to 10% ceAF for 48 h and cell supernatant was collected for testing. Secreted IL-6, IL-10, TGF-β1, and TNF-α were measured with ELISA kit according to the kit instructions (Elabscience, China).

### Statistical analysis

Experimental data were analyzed using Graphpad Prism v8.0 and expressed as Mean ± SEM. Parametric test was applied to data that follow the normal distribution (verified via the Shapiro-Wilk test). If the data were normally distributed, statistic differences between multiple groups were compared using one-way ANOVA and Newman-Keuls post hoc test. If the data passed the normality test, the two groups were compared using the two-tailed student's t-test. The composite effect of two factors were analyzed using two-way ANOVA, followed by Tukey's post hoc test. At least three independent determinations were conducted and P<0.05 was defined as statistically significant.

### Embodiment 1: ceAF alleviating inflammation and oxidative stress in lipopolysaccharide (LPS)-stimulated RAW 264.7 cells via TLR4/NF-κB signaling pathway

To evaluate the regulatory effect of ceAF on lipopolysaccharide (LPS)-induced cellular inflammation, RAW264.7 cells were used as experimental cells in this embodiment, and LPS (100 ng/ml) stimulation was performed for 48 h to induce cellular inflammation.

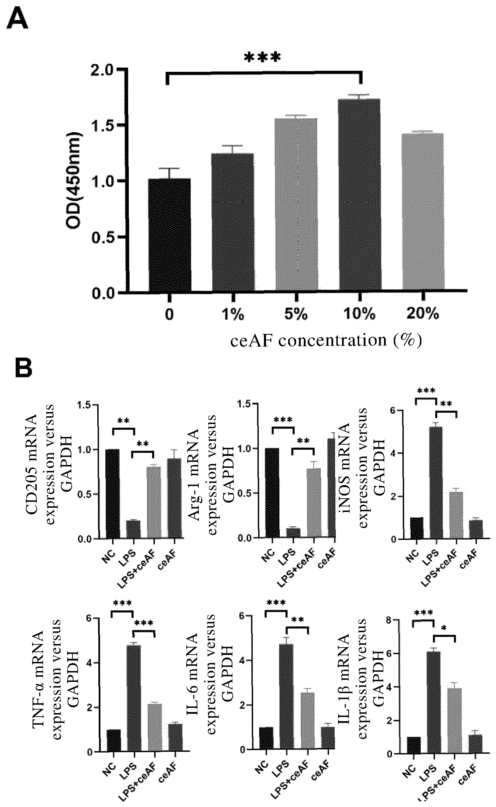

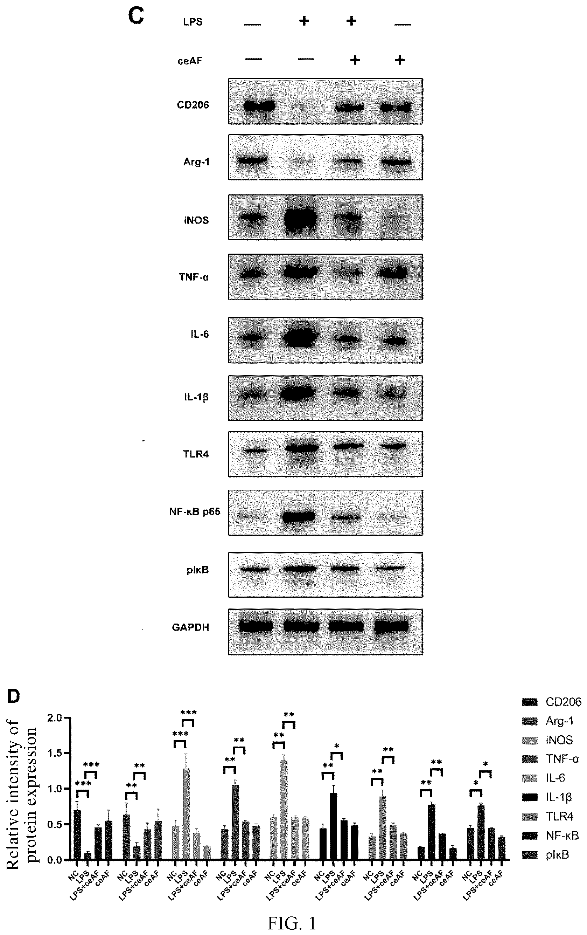

Firstly, the cell proliferation and viability were detected with different concentrations of ceAF. CCK-8 results showed that the proliferation and viability of RAW264.7 cells enhanced with the increase of ceAF concentration, reaching the peak at a concentration of 10% (FIG.1, A). Then RAW264.7 cells were divided into blank control group (NC), LPS stimulation group (LPS), LPS and ceAF combined intervention group (LPS + ceAF), and ceAF intervention group (ceAF). The qPCR results indicated that decreased M2 macrophage-related gene (CD206), increased M1 macrophage-related gene (iNOS), and upregulated inflammation-related genes (TNF-α, IL-6, IL-1β) at the transcription level after LPS stimulation. These trends were reversed after ceAF intervention (FIG.1, B). Western blotting (WB) experiments at the translation level were further performed, with the same trends obtained (FIG.1, C-D).

### Embodiment 2: ceAF inducing polarization of RAW264.7 cells toward M2 macrophages in vitro

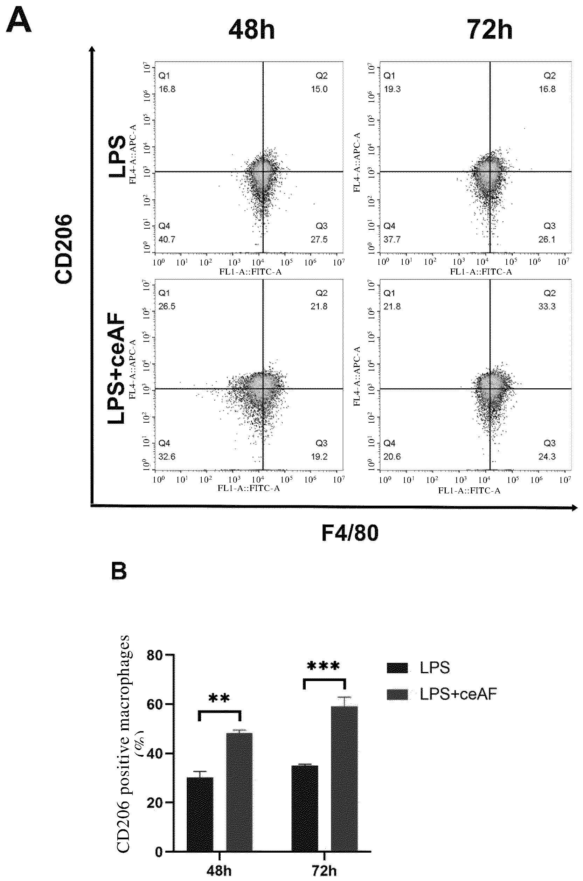

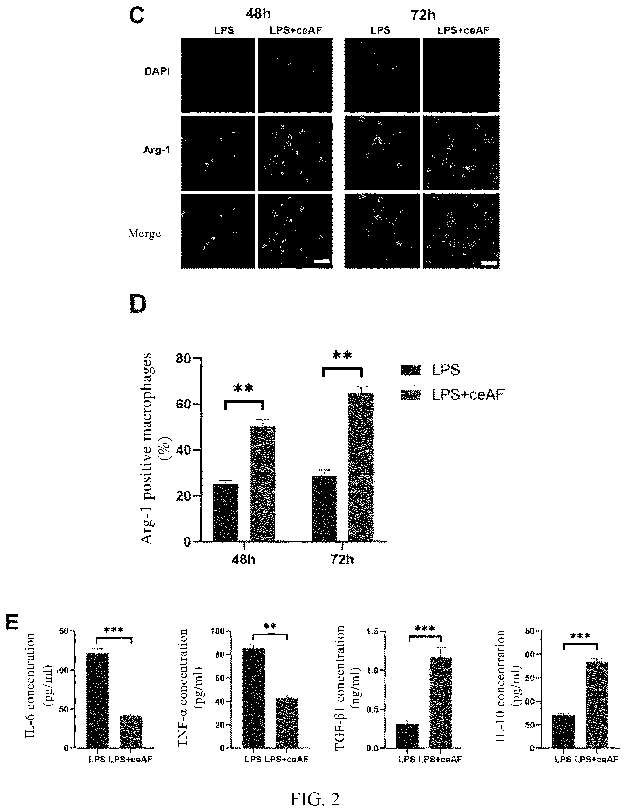

The ceAF-induced polarization of macrophages toward M2 was verified using flow cytometry and cellular immunofluorescence. In terms of flow cytometry, CD86 was used to label macrophages and CD206 locate M2 macrophages. In terms of cellular immunofluorescence, Arg-1 was used to locate M2 macrophages. The results showed that ceAF intervention effectively increased the proportion of M2 macrophages at both 48 h and 72 h (FIG. 2, A-D). Supernatants of RAW264.7 cells were collected and ELISA was used to detect inflammation-related cytokines. The results showed that pro-inflammatory cytokines IL-6 and TNF-α significantly decreased after ceAF intervention, and M2-related secretory cytokines TGF-β1 and IL-10 significantly increased after ceAF intervention (FIG.2, E).

### Embodiment 3: ceAF promoting wound healing in STZ-induced diabetic mice

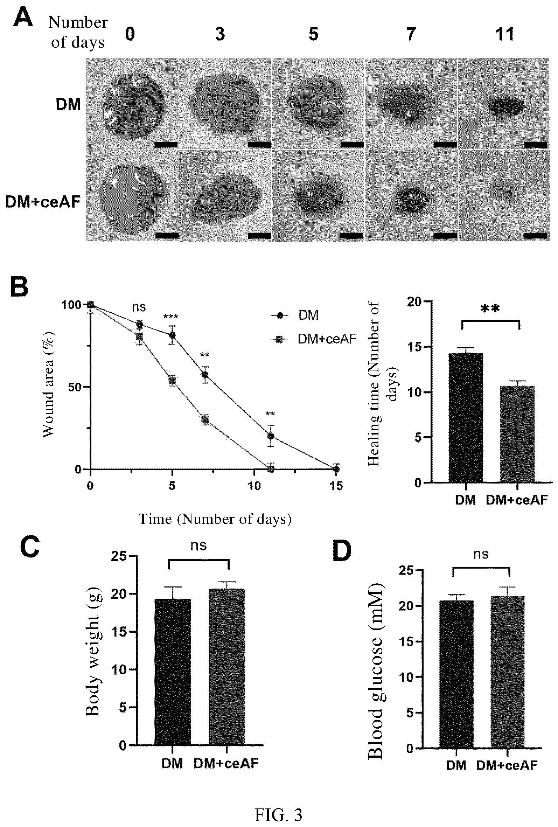

To verify the effect of ceAF on the wound healing of streptozotocin (STZ)-induced diabetic mice, 8-week-old male mice were divided into two groups. STZ (50 mg/kg) was injected into the abdominal cavities of mice for five consecutive days. The blood glucose was measured one week later, and the diabetes modeling was deemed successful if the blood glucose was greater than 16.7 mM. A full-thickness wound with a diameter of 8 mm were made in a sharp manner on the central dorsal skin of a mouse with surgical scissors. The control group (DM group) was treated with conventional dressing change every day, and the experimental group was treated with 10% ceAF topical dressing change every day. Wound photos were shot using a camera on Day 0, Day 3, Day 5, Day 7, and Day 11. The results indicated that the healing speed of ceAF group was significantly faster than that of control group from the 5th day (FIG.3, A-B). There was no significant difference in body weight and blood glucose measured on the 11th day between the two groups (FIG. 3, C-D), thus the two confounding factors of body weight and blood glucose could be excluded.

### Embodiment 4: ceAF improving histological parameters of wounds in STZ-induced diabetic mice

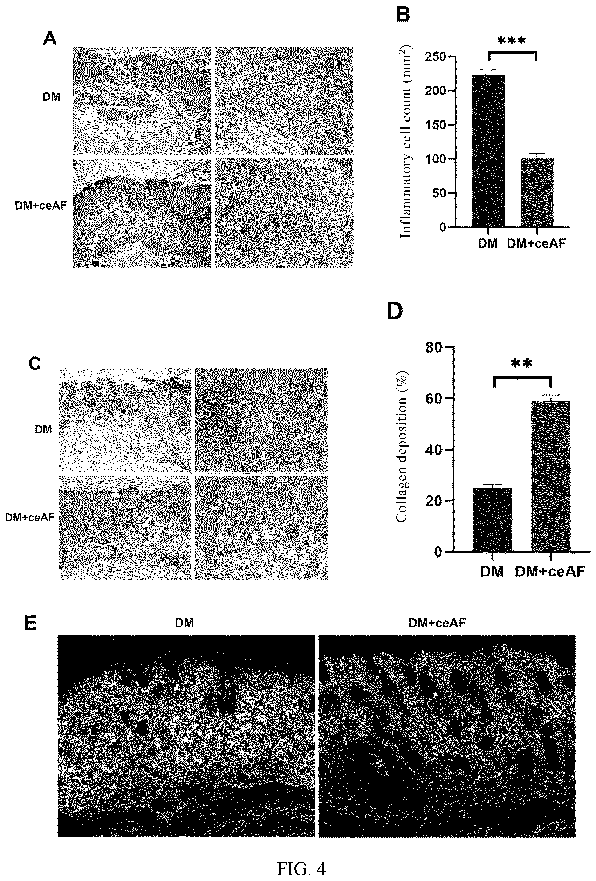

This embodiment further confirmed the effect of ceAF on the wounds in STZ-induced diabetic mice through histopathological analysis. H&E staining was performed to observe the inflammatory cell infiltration in wound margin tissues on the 5th day after wound modeling. It was found that inflammatory cells in the ceAF group significantly reduced (FIG.4, A-B). On the 10th day after wound modeling, MT staining was performed to observe the collagen deposition in wound margin tissues. It was found that collagen deposition in the ceAF group significantly increased (FIG.4, C-D). After the wounds in both groups healed, the skin tissues in the healing areas were taken for sirius red staining to evaluate the healing quality (FIG.4, E). The results showed predominent type III collagen and type I collagen in the ceAF group and the control group respectively, indicating that scars formed in the ceAF group was less and the healing quality was better.

### Embodiment 5: ceAF improving wound healing-related indicators of STZ-induced diabetic mice

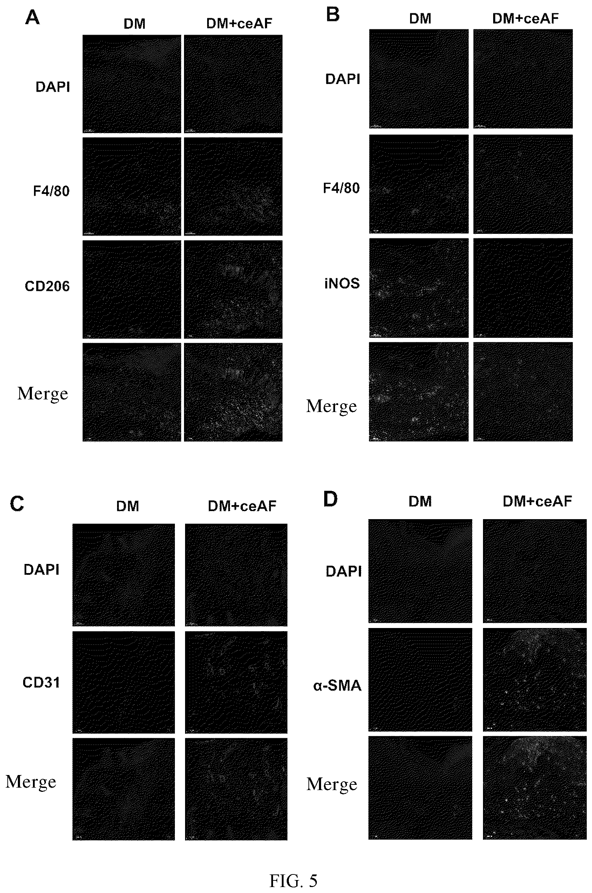

The tissue immunofluorescence further confirmed the effect of ceAF on the wound healing-related indicators of STZ-induced diabetic mice. The skin wound margin tissues on the 5th day were taken, with the co-staining of F4/80 and CD206 performed to locate M2 macrophages and that of F4/80 and iNOS performed to locate M1 macrophages. It was found that the number of M2 macrophages significantly increased (FIG.5, A) and that of M1 macrophages significantly decreased (FIG.5, B) at wound margins of the ceAF group. Skin wound margin tissues on the 10th day were taken and the co-staining of CD31 and α-SMA was performed (FIG.5, C-D). The results indicated a significant increase in neovascularization in the ceAF group.

### Embodiment 6: ceAF regulating wound-related inflammation cytokines in STZ-induced diabetic mice

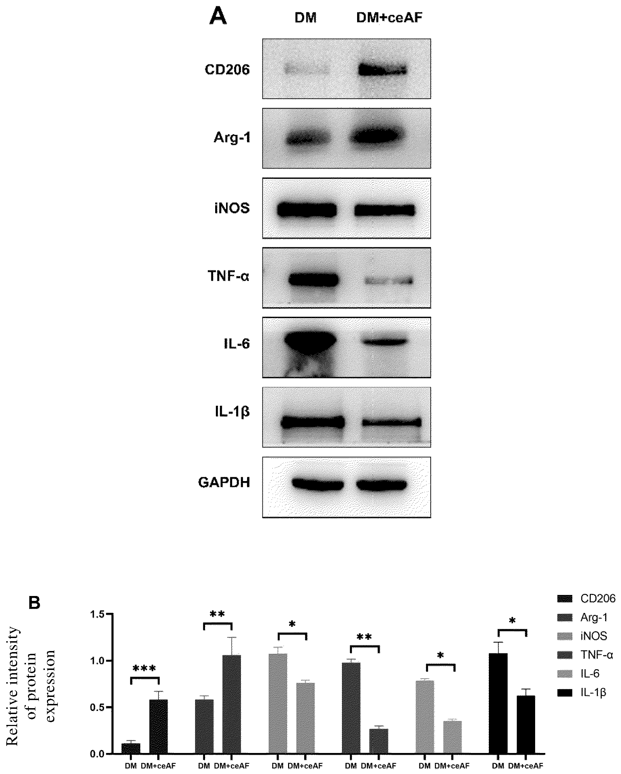

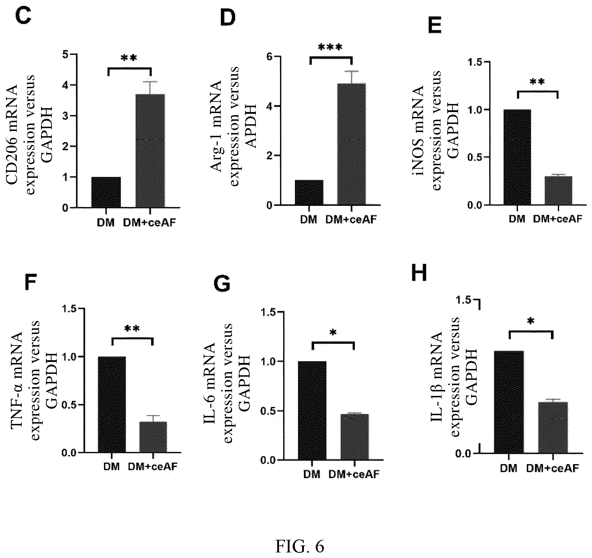

To verify the effect of ceAF on the wound-related inflammation cytokines in STZ-induced diabetic mice *in vivo,* the wound margin tissues on the 5th day after wound modeling were taken for performing WB and qPCR detections. It was found that in the ceAF group, the transcription and translation levels of M2 macrophage genes CD206 and Arg-1 were increased (FIG.6, A, C-D), and the transcription and translation levels of M1 macrophage marker gene iNOS as well as inflammation-related genes TNF-α, IL-6, and IL-1β were decreased (FIG.6, A, E-H).

In conclusion, the results indicated that ceAF could improve the LPS-induced inflammatory responses of RAW264.7 cells *in vitro* and facilitate the wound healing of STZ-induced diabetic mice *in vivo.* This function is achieved by regulating the TLR 4/NF-κB signaling pathway.

## Claims

1. The use of amniotic fluid in the preparation of medicines for treating and/or preventing macrophage-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

2. The use of amniotic fluid in the preparation of medicines for treating and/or preventing M1 macrophage-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

3. The use of amniotic fluid in the preparation of medicines for treating and/or preventing M2 macrophage-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

4. The use of amniotic fluid in the preparation of formulations for treating and/or preventing TLR4/NF-κB signaling pathway-mediated diseases; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

5. The use of amniotic fluid in the preparation of formulations for inhibiting the number of M1 macrophage populations and increasing the proportion of M2 macrophages, or in the preparation of formulations for promoting the transformation of M1 macrophages into M2 macrophages; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

6. The use of any one of claims 1 to 5, wherein the medicine or formulation is a cell culture containing the amniotic fluid and/or embryonic stem cells.
Preferably, the medicine or formulation is a pharmaceutical composition containing the amniotic fluid and/or chicken embryonic stem cells along with pharmaceutically acceptable excipients.

7. The use of claim 1, wherein the macrophage-mediated diseases are selected from: hypertrophic scars; chronic obstructive pneumonia; tumors, such as breast cancer, liver cancer; metabolic diseases, such as severe obesity, insulin resistance, type II diabetes; inflammatory diseases, such as acute pancreatitis, atherosclerosis; cardiovascular diseases, such as myocarditis, myocardial infarction, cardiac arrhythmia; neuropathies, such as Alzheimer's disease; cerebral diseases, such as cerebral infarction, cerebral injury; ophthalmic diseases, such as autoimmune uveitis, retinopathy, keratitis, corneal transplantation, sicca syndrome, uveal melanoma, myopia; immune inflammatory diseases, such as inflammatory enteritis, autoimmune hepatitis, asthma, alcoholic liver disease, colitis, multiple sclerosis, periodontitis; arthritis, such as rheumatoid arthritis, bone erosion, synovitis, osteoarthritis; nephritides, such as acute kidney injury, chronic kidney disease, end-stage renal disease, proliferative glomerulonephritis, membranous nephropathy, diabetic nephropathy, Henoch-Schönlein purpura nephritis, ANCA-associated vasculitis, urinary tract infection, autosomal dominant polycystic kidney disease; bacterial infectious diseases, such as sepsis; gestational hypertension; diabetes, gestational diabetes, diabetic nephropathy.

8. The use of claim 2, wherein the M1 macrophage-mediated diseases are selected from: hypertrophic scars in regression phase; chronic obstructive pneumonia; tumors, such as breast cancer, liver cancer; metabolic diseases, such as severe obesity, insulin resistance, type II diabetes; inflammatory diseases, such as acute pancreatitis; coronary artery diseases, such as atherosclerosis; nephropathy; obesity; cardiovascular diseases, such as myocarditis, myocardial infarction; cerebral diseases, such as cerebral infarction, cerebral injury; ophthalmic diseases, such as autoimmune uveitis, retinopathy, keratitis, corneal transplantation, sicca syndrome, uveal melanoma; immune inflammatory diseases, such as inflammatory enteritis, autoimmune hepatitis, asthma, alcoholic liver disease, colitis, multiple sclerosis, periodontitis, osteoarthritis; diabetes, gestational diabetes, diabetic nephropathy.

9. The use of claim 3, wherein the M2 macrophage-mediated diseases are selected from: hypertrophic scars in proliferative phase; tumors, such as breast cancer, liver cancer; metabolic diseases, such as insulin resistance, type II diabetes; inflammatory diseases, such as acute pancreatitis; cardiovascular diseases, such as myocardial infarction, myocardial failure, atherosclerosis, coronary artery disease, myocarditis; cerebral infarction; ophthalmic diseases, such as autoimmune uveitis, retinopathy, keratitis, corneal transplantation, sicca syndrome, uveal melanoma; immune inflammatory diseases, such as inflammatory enteritis, autoimmune hepatitis, asthma, alcoholic liver disease, colitis, multiple sclerosis, periodontitis, osteoarthritis; diabetes, gestational diabetes, diabetic nephropathy.

10. The use of claim 4, wherein the TLR4/NF-κB signaling pathway-mediated diseases are selected from: systemic lupus erythematosus; vascular inflammations, such as atherosclerosis, coronary heart disease; myocarditis, such as myocardial ischemic tissue inflammation, myocardial injury; hepatitis, such as hepatic failure, alcoholic liver injury, inflammatory immune responses during alcohol metabolism; fatty liver; pneumonia, such as acute lung injury, chronic obstructive pulmonary disease, silicosis; nephritis, such as acute kidney injury, lupus nephritis; inflammatory bowel diseases, such as acute enteritis, ulcerative colitis, radiation proctitis; gastritis, such as chronic atrophic gastritis; acute respiratory infections, such as pneumonia, bronchitis, pharyngitis, sinusitis, otitis media; periodontitis, hyperuricemia; rhinallergosis, allergic rhinitis; mastitis; arthritis, such as acute gouty arthritis, chronic arthritis, rheumatoid arthritis; wound tissue inflammation, hypertrophic scars; polycystic ovary syndrome; tumors, such as pituitary prolactinoma, adrenocorticotropic hormone adenoma, intracranial aneurysm; infectious diseases, such as bacterial infections, fungal infections, viral infections; allergic diseases, such as allergic skin diseases, bronchial asthma, allergic rhinitis, Henoch-Schönlein purpura.
